# EUROPEAN PATENT APPLICATION

(11) **EP 4 691 403 A1**
(43) Date of publication of application: **11.02.2026**
(21) Application number: 24306327.8
(22) Date of filing: 06.08.2024
(51) Int. Cl.: A61B 34/10, A61B 8/00, A61B 34/00, A61B 17/00

(54) **SUPPORT TOOL FOR PLANNING USE OF SURGICAL MESH FOLLOWING LAPAROTOMY OR VENTRAL HERNIA REPAIR**

(71) Applicant: Sofradim Production, 01600 Trevoux (FR)
(72) Inventor: JOURDAN, Arthur, 01600 Trevoux (FR); LE RUYET, Anicet, 01600 Trevoux (FR); BLANC, Marion, 01600 Trévoux (FR)
(74) Representative: Germain Maureau

(57) **Abstract**

A system for pre-operative decision support includes a memory and a processor. The memory is configured to store a minimum threshold shear modulus or shear modulus range for abdominal tissue of a patient. The processor is configured to execute one or more programs to perform a plurality of operations. These operations include receiving at least one shear modulus measurement acquired by shear wave elastography (SWE) that is representative of a shear modulus of abdominal tissue of a patient within a predetermined distance of a planned abdominal incision, comparing the at least one shear modulus measurement with the minimum threshold shear modulus or shear modulus range, and outputting a recommendation regarding use of a surgical mesh following a laparotomy based upon the comparison.

## Description

### BACKGROUND

In general, a laparotomy is a surgical procedure that opens a patient's abdomen to expose their organs. An incision is made through the patient's abdominal wall to allow access to the peritoneal cavity, which includes the abdomen and pelvis. Laparotomies can be employed in exploratory procedures to directly examine the patient's condition, as well as to take tissue samples to diagnose the patient's condition. Laparotomies can also be employed in therapeutic procedures to remove an organ, manage a critical condition, or find and fix a problem during an exploratory procedure.

A ventral hernia is a hernia that occurs through the front abdominal muscles. A hernia occurs when one of the organs pushes through the muscle or tissue that contains it. Ventral hernias can include primary hernias and incisional hernias. Primary hernias commonly occur in the body midline at the abdominal region to weakness in the abdominal wall anatomy. Ventral hernia repair can be performed by making an incision in the abdominal wall and gently pushing the contents of the hernia back into the abdomen.

Incisional hernias are a type of complication that can arise following a laparotomy or ventral hernia repair. An incisional hernia occurs when an organ, intestine, or fatty tissue squeezes through the abdominal incision made during a laparotomy or the primary closure of a ventral hernia defect.

### SUMMARY

Surgical mesh is a medical device that can be implanted in a patient within the vicinity of an incision to provide additional support during recovery. However, a surgeon must make a variety of decisions when employing the surgical mesh following a laparotomy. Examples of such decisions can include, but are not limited to, a type of surgical mesh, a size and shape of the surgical mesh, placement of the surgical mesh, and how to secure (fixate) the mesh in place with respect to surrounding tissue, amongst others. Should the surgeon make an error in one or more of these decisions, the efficacy of the surgical mesh to inhibit occurrence of an incisional hernia following a laparotomy or ventral hernia repair can be significantly reduced.

Embodiments of the present disclosure provide systems and methods for pre-operative decision support regarding use of surgical mesh after a planned laparotomy or ventral hernia repair. In an embodiment, a system for pre-operative decision support (102) is provided. The system includes a memory (120) and a processor (122). The memory (120) is configured to store a minimum threshold shear modulus or shear modulus range for abdominal tissue (116) of a patient (200). The processor (122) is in communication with the memory (120) and is configured to execute one or more programs to perform plurality of operations. The plurality of operations includes receiving at least one shear modulus measurement acquired by shear wave elastography (SWE) that is representative of a shear modulus of abdominal tissue (116) of the patient (200) within a predetermined distance D of a planned abdominal incision (204). The plurality of operations further includes comparing the at least one shear modulus measurement with the minimum threshold shear modulus or shear modulus range. The plurality of operations additionally includes outputting a recommendation regarding use of a surgical mesh (306) following a laparotomy based upon the comparison.

In an additional embodiment, the one or more programs executed by the processor (122) are further configured to perform operations including outputting a recommendation to use a surgical mesh (306) when the at least one shear modulus measurement is less than the minimum threshold shear modulus or lies outside of the shear modulus range.

In an additional embodiment, the at least one shear modulus measurement is an average of a plurality of shear modulus measurements acquired at different locations of the patient's abdomen (202) within the predetermined distance D from the planned abdominal incision (204).

In an additional embodiment, the at least one shear modulus measurement includes a plurality of shear modulus measurements acquired at different locations of the patient's abdomen (202) within the predetermined distance D from the planned abdominal incision (204), and the one or more programs executed by the processor (122) are configured to perform a plurality of further operations. The further operations include identifying abdominal locations at which the at least one shear modulus measurement is less than the minimum threshold shear modulus (304) or lies outside of the shear modulus range. The further operations also include determining a shape and size for a surgical mesh (306) that covers each of the identified abdominal locations and the planned abdominal incision. The further operations additionally include outputting the determined size and shape.

In an additional embodiment, the one or more programs executed by the processor (122) are configured to perform a plurality of further operations. The further operations include receiving an optical image of the abdomen (202) of the patient (200) within the predetermined distance D of the planned abdominal incision (204). The further operations also include generating a composite image (300) that overlays, upon the optical image, graphical objects representing the planned abdominal incision (204), the determined size and shape of the surgical mesh (306), and at the least one shear modulus measurement (302, 304). The further operations additionally include outputting the generated composite image (300) for display on a display device (114).

In an additional embodiment, the memory (120) is further configured to store a plurality of fixation techniques for securing a surgical mesh (306) to abdominal tissue of a patient (200) and a range of shear modulus values corresponding to each of the plurality of fixation techniques, and the one or more programs executed by the processor (122) are configured to perform further operations. The further operations include identifying the modulus range that includes the at least one shear modulus measurement. The further operations additionally include selecting the fixation technique from the plurality of fixation techniques that corresponds to the identified shear modulus range. The further operations also include outputting the selected fixation technique.

In an additional embodiment, the memory (122) is further configured to store information for each of a plurality of surgical meshes, the information including a shear modulus, and the one or more programs executed by processor (122) are configured to perform further operations. The further operations include selecting a surgical mesh (306) from the plurality of surgical meshes based upon a comparison of the at least one shear modulus of the plurality of surgical meshes with the at least one shear modulus measurement. The further operations also include outputting the selected surgical mesh (306).

In an additional embodiment, the selected surgical mesh (306) has a shear modulus greater than the minimum threshold shear modulus or lies within the shear modulus range

In an additional embodiment, the at least one shear modulus measurement includes a first shear modulus measurement representative of the shear modulus of the patient's abdominal tissue in a first direction and a second shear modulus measurement representative of the shear modulus of the patient's abdominal tissue in a second direction, different from the first direction. The information stored by the memory (120) also includes a shear modulus in the first direction and the second direction of each surgical mesh, and the one or more programs executed by the processor (122) are configured to perform operations. The further operations include selecting the surgical mesh (306) from the plurality of surgical meshes based upon a comparison of the first shear modulus measurement in the first direction with the shear modulus of the plurality of meshes in the first direction and a comparison of the first shear modulus measurement in the second direction with the shear modulus of the plurality of meshes in the second direction.

In an additional embodiment, the selected surgical mesh (306) has a shear modulus in the first and second directions greater than the minimum threshold shear modulus or lies within the shear modulus range.

In an embodiment, a method (400) for pre-operative decision support is provided. The method (400) includes receiving, by a processor (122) from a memory (120), a minimum threshold shear modulus or shear modulus range for abdominal tissue of a patient (200). The method (400) further includes receiving, by the processor (122), at least one shear modulus measurement acquired by shear wave elastography (SWE) that is representative of a shear modulus of abdominal tissue of the patient (202) within a predetermined distance of a planned abdominal incision. The method (400) additionally includes comparing, by the processor (122), the at least one shear modulus measurement with the minimum threshold shear modulus or shear modulus range. The method (400) also includes outputting by the processor (122), a recommendation regarding use of a surgical mesh (306) following a laparotomy based upon the comparison.

In an additional embodiment, the method (400) further includes, by the processor (122), outputting a recommendation to use a surgical mesh (306) when the at least one shear modulus measurement is less than the minimum threshold shear modulus or lies outside of the shear modulus range.

In an additional embodiment, the at least one shear modulus measurement is an average of a plurality of shear modulus measurements acquired at different locations of the patient's abdomen (202) within the predetermined distance D from the planned abdominal incision (204).\

In an additional embodiment, the at least one shear modulus measurement includes a plurality of shear modulus measurements acquired at different locations of the patient's abdomen (202) within the predetermined distance D from the planned abdominal incision (204). The method (400) further includes by the processor (122), identifying abdominal locations at which the at least one shear modulus measurement is less than the minimum threshold shear modulus or lies outside of the shear modulus range, determining a shape and size for a surgical mesh (306) that covers each of the identified abdominal locations and the planned abdominal incision, and outputting the determined size and shape.

In an additional embodiment, the method (400) further includes, by the processor (122), receiving an optical image of the abdomen (202) of the patient (200) within the predetermined distance D of the planned abdominal incision (204), generating a composite image that overlays, upon the optical image, graphical objects representing the planned abdominal incision (204), the determined size and shape of the surgical mesh (306), and at the least one shear modulus measurement (302, 304), and outputting the generated composite image (300) for display on a display device (114).

In an additional embodiment, the method (400) further includes, by the processor (122), receiving a plurality of fixation techniques for securing a surgical mesh (306) to abdominal tissue of a patient (200) and a range of shear modulus values corresponding to each of the plurality of fixation techniques, identifying the modulus range that includes the at least one shear modulus measurement, selecting the fixation technique from the plurality of fixation techniques that corresponds to the identified shear modulus range, and outputting the selected fixation technique.

In an additional embodiment, the method (400) further includes, by the processor (122), receiving information for each of a plurality of surgical meshes, the information including a shear modulus, selecting a surgical mesh (306) from the plurality of surgical meshes based upon a comparison of the at least one shear modulus of the plurality of surgical meshes with the at least one shear modulus measurement, and outputting the selected surgical mesh (306).

In an embodiment, the selected surgical mesh (306) has a shear modulus greater than the minimum threshold shear modulus or lies within the shear modulus range.

In an embodiment, the at least one shear modulus measurement includes a first shear modulus measurement representative of the shear modulus of the patient's abdominal tissue in a first direction and a second shear modulus measurement representative of the shear modulus of the patient's abdominal tissue in a second direction, different from the first direction. The method (400) further includes, by the processor (122), receiving a shear modulus in the first direction and the second direction of each surgical mesh and selecting the surgical mesh (306) from the plurality of surgical meshes based upon a comparison of the first shear modulus measurement in the first direction with the shear modulus of the plurality of meshes in the first direction and a comparison of the first shear modulus measurement in the second direction with the shear modulus of the plurality of meshes in the second direction.

In an embodiment, the selected surgical mesh (306) has a shear modulus in the first and second directions greater than the minimum threshold shear modulus or lies within the shear modulus range.

### DESCRIPTION OF DRAWINGS

These and other features will be more readily understood from the following detailed description taken in conjunction with the accompanying drawings, in which:
FIG. 1 is a schematic diagram illustrating an operating environment including an exemplary embodiment of a pre-operative decision support system;
FIG. 2 is a schematic diagram illustrating a patient's abdomen;
FIG. 3 is a schematic diagram illustrating a composite image including an image of a patient's abdomen and overlaid graphical objects; and
FIG. 4 is a flow diagram illustrating one exemplary embodiment of a method for pre-operative decision support.

It is noted that the drawings are not necessarily to scale. The drawings are intended to depict only typical aspects of the subject matter disclosed herein, and therefore should not be considered as limiting the scope of the disclosure.

### DETAILED DESCRIPTION

Embodiments of the present disclosure provide systems and methods for pre-operative decision support regarding use of surgical mesh after a planned laparotomy or ventral hernia repair. As discussed in greater detail below, in one aspect, these systems and methods can generate a recommendation for whether a surgical mesh should be used in a subject circumstance. The recommendation can be made using measurements of shear modulus of abdominal tissue by shear wave elastography (SWE). SWE uses acoustic (sound) waves to measure the elastic properties of the patient's tissues (e.g., abdominal tissue), such as shear modulus and identify abnormally weak regions of the patient's tissue that may indicate higher risk of developing an incisional hernia or recurrent hernia (post-hernia repair) and may benefit from the use of surgical mesh to inhibit development or recurrence of an incisional hernia. In another aspect, when the use of surgical mesh is recommended, these systems and methods can further use the shear modulus measurements from the SWE to select a specific type of mesh, one or more parameters of the mesh, such as size, shape, or anisotropy, and/or a manner of fixation of the mesh to tissue. In this manner, pre-operative decisions regarding use of mesh can be facilitated. By tailoring the pre-operative decisions to the elastic properties of the patient, rather than average elastic properties of demographic matching the patient, surgeon error can be prevented, improving the likelihood of positive outcomes of laparotomy and ventral hernia repair, such as restoration of physiological behavior within the vicinity of a planned abdominal incision and/or avoiding development/recurrence of an incisional hernia.

While the present technology is discussed in the context of abdominal tissue, embodiments of the disclosure can be employed with any tissue without limit.

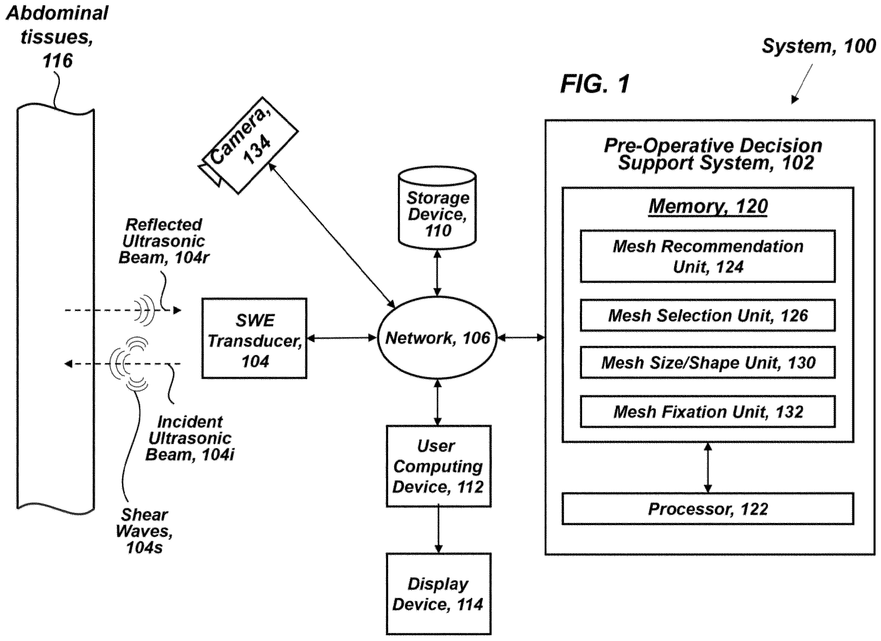

FIG. 1 is a diagram illustrating an embodiment of a system 100 including a pre-operative support system 102. As shown, the system 100 includes a shear wave elastography (SWE) transducer 104 in communication with a network 106 (e.g., LAN, WAN, Internet, etc.) The network 106 is further in communication with a storage device 110, a user computing device 112, and the pre-operative decision support system 102, also referred to herein as a decision support system 102. Each of the components of the system 100 and operation of the decision support system 102 is discussed in greater detail below. It may be understood that, while the components of FIG. 1 are illustrated in communication via the network 106, in alternative embodiments, any one or more of these components may communicate with another directly without the network 106.

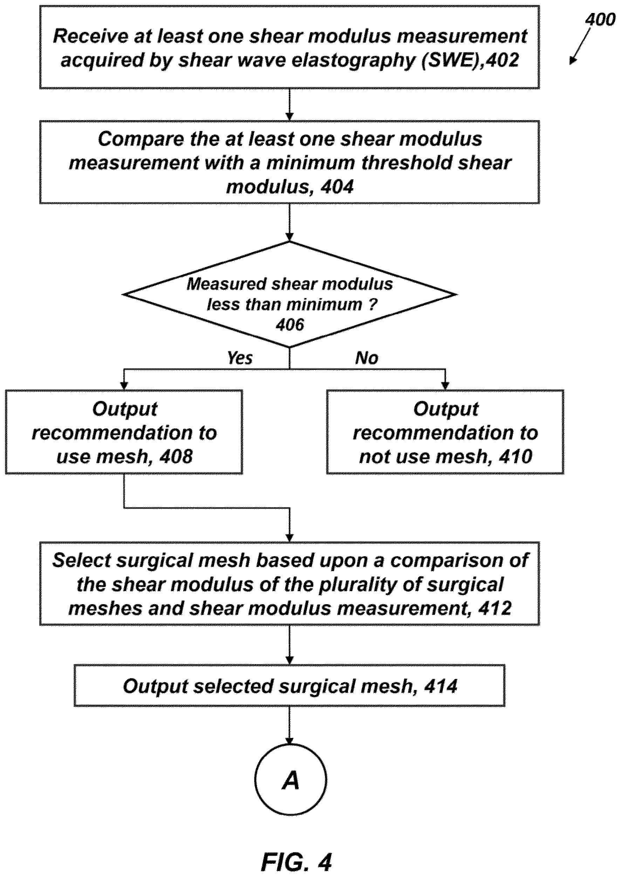

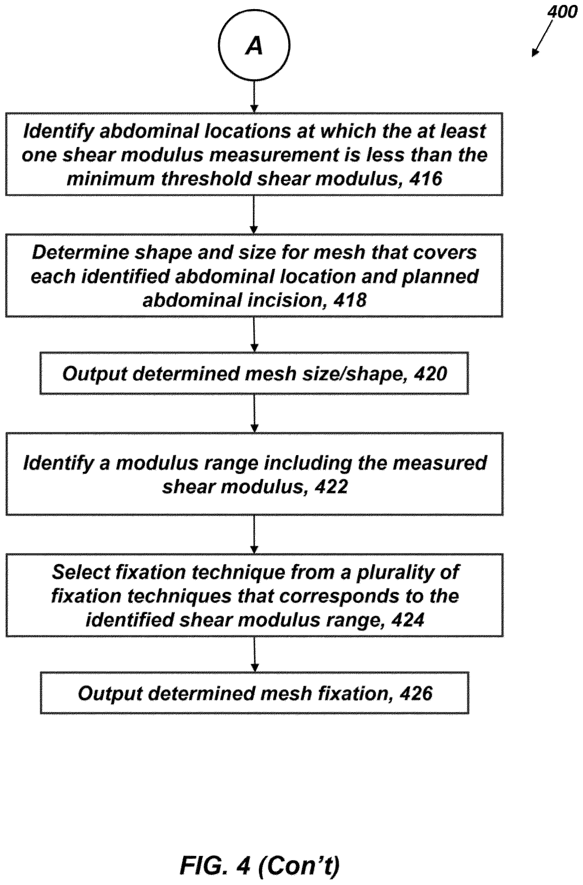

A corresponding method 400 for pre-operative decision support is illustrated in FIG. 4 and includes operations 402-426. However, it can be appreciated that alternative embodiments of the method can include greater or fewer operations and the order of the operations can be different than that illustrated in FIG. 4.

The SWE transducer 104 is configured to emit an ultrasonic beam 104i that is incident upon the patient's abdominal tissues 116 and to detect ultrasonic beams 104r reflected from abdominal tissues 116 (e.g., linea alba and abdominal muscles such as rectus abdominis, external oblique, internal oblique, transversus abdominis). The incident ultrasonic beam 104i includes shear waves 104s that travel perpendicular to a direction of propagation of the incident beam 104i. Ultrasonic beams 104r reflected from the abdominal tissue 116 can be used to detect displacement of the abdominal tissue 116 responsive to the incident ultrasonic beam 104i for calculation of shear wave velocity in the abdominal tissue 116 and abdominal tissue shear modulus.

The measured shear wave velocity and abdominal tissue shear modulus can be stored in the storage device 110. The storage device 110 can be any device capable of storing computer-readable data. Examples include, but are not limited to, hard disk drives (HDDs), solid state drives (SSDs), universal serial bus (USB) drives, memory cards, network-attached storage (NAS) devices, and the like. As discussed herein, it can be understood that measurements of abdominal tissue shear wave velocity and shear modulus are further correlated to a position at which incident ultrasonic beam is incident upon the patient. The position is provided in a predetermined coordinate system.

The decision support system 102 can receive the measured shear wave velocity and abdominal tissue shear modulus directly from the SWE transducer 104 in real-time or from the storage device 110 after storage (method 400, operation 402). In general, regions of abdominal tissue 116 exhibiting abnormal shear wave velocity and/or attendant abnormal shear modulus can be indicative of higher likelihood of developing incisional hernias. As discussed below, reference is made to shear modulus in the context of the decision support system 102. However, in alternative embodiments, corresponding shear velocities can be used in lieu of, or in combination with, shear modulus by the decision support system 102.

Thus, the shear wave velocity and shear modulus measurements as a function of position can be used by the decision support system 102 to determine whether a surgical mesh should be used in connection with a planned surgery, such as a laparotomy and, if so, to further assist with selection of a suitable surgical mesh. In the case of ventral hernia repair, where mesh is typically used by default, the decision support system 102 can further assist with selection of a suitable surgical mesh.

The decision support system 102 can include a memory 120 in communication with a processor 122. The processor 122 can execute a plurality of operations to perform various functions, represented as units (e.g., programs) stored in the memory 120. As shown, the units can include a mesh recommendation unit 124, a mesh selection unit 126, a mesh size/shape unit 130, and a mesh fixation unit 132.

The memory 120 can include a variety of information employed by the processor 122 to perform the operations of the respective units, discussed in greater detail below. The information stored by the memory 120 can be received from a variety of different sources. In one aspect, the information can be received by the memory 120 from user input via a user computing device 112 (e.g., a desktop computer, laptop computer, smart phone, tablet computing device, or the like). In another aspect, the information can be retrieved by the memory 120 from the storage device 110 or another storage device on another network (not shown).

The mesh recommendation unit 124 can a program stored in the memory 120 and executed by the processor 122 to determine whether to recommend use of a surgical mesh following a laparotomy. The SWE measurements retrieved by the decision support system 102 can include shear wave velocities in the abdominal tissue 116 and/or abdominal tissue shear modulus as a function of position within the abdomen. The memory 120 can store a minimum threshold shear modulus for the abdominal tissue 116 of a patient (a minimum shear modulus for healthy abdominal tissue). The memory 120 can also store a proposed location for a planned abdominal incision. The minimum threshold shear modulus represents a shear modulus below which the abdominal tissue is considered at elevated risk for incisional hernia and at or above which the abdominal tissue is considered to have a normal risk for incisional hernia. Alternatively or additionally, the memory 120 can store a shear modulus range extending from the minimum shear modulus to a maximum shear modulus, representing a range of values for healthy abdominal tissue.

The mesh recommendation unit 124 can determine at least one shear modulus measurement representative of the abdominal tissue 116 within a predetermined distance D of the planned abdominal incision 204. In one aspect, the shear modulus measurement can be an average (e.g., a statistical mean, median, or mode) of all received shear modulus measurements within the predetermined distance D. In another example, the abdomen 116 can be divided into different abutting zones of approximately equal area and the at least one shear modulus measurement can be a difference between shear modulus measurements of neighboring abdominal zones. The difference can be a difference between minimum modulus measurements of neighboring zones, a difference between maximum modulus measurements of neighboring zones, a difference between minimum and maximum modulus measurements of neighboring zones, etc. Such differences can be useful in identifying weak and strong areas of the abdomen, as well as areas of "instability" where the abdomen 116 is atypically weak.

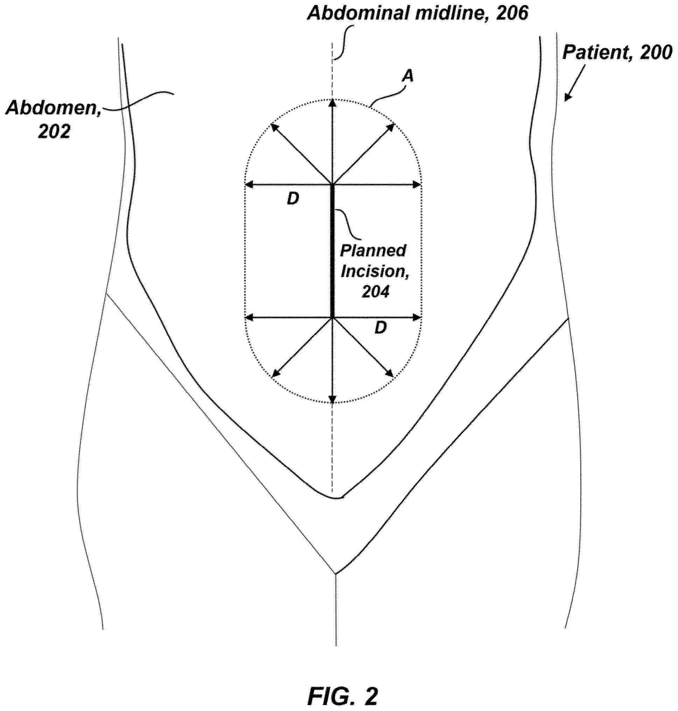

For example, FIG. 2 illustrates a patient 200 including abdomen 202 along with a planned abdominal incision 204 at an abdominal midline 206. Further illustrated is an area A (dashed line envelope) within the predetermined distance D of the planned abdominal incision 204.

In an embodiment, the mesh recommendation unit 124 can compare the at least one shear modulus measurement with the minimum threshold shear modulus or shear modulus range (method 400, operation 404-406). A recommendation regarding whether to use a surgical mesh following a laparotomy can be further output based upon this comparison. For example, when the at least one shear modulus measurement is determined to be less than the minimum threshold shear modulus or lies outside of the shear modulus range, the mesh recommendation unit 124 can output a recommendation to use a surgical mesh (method 400, operation 408). Conversely, when the at least one shear modulus measurement is determined to be greater than or equal to the minimum threshold shear modulus or lies within the shear modulus range, the mesh recommendation unit 124 can output a recommendation not to use a surgical mesh (method 400, operation 410).

Output from any of the units 124-132 of the decision support system 102 can be performed in a variety of ways. In one aspect, an output can be stored in the memory 120 and/or the storage device 110. A user, employing the user computing device 112, can subsequently retrieve the recommendation for display on a display device 114. Alternatively, the output can be transmitted to the user computing device 112 and displayed on the display device 114 following its generation.

When the mesh recommendation unit 124 recommends use of a surgical mesh for a a planned laparotonry, or when planning ventral hernia repair, the mesh selection unit 126 can select at least one suitable surgical mesh (method 400, operation 412). For example, the memory 120 can further store shear modulus information for each of a plurality of surgical meshes. In one aspect, the mesh selection unit 126 can select at least one surgical mesh from the plurality of surgical meshes that has a shear modulus greater than the minimum threshold shear modulus.

In other aspects, when the at least one selected surgical mesh includes a plurality of surgical meshes, a user can request the decision support system 102 to filter the plurality of selected surgical meshes (e.g., using the user computing device 112) based upon one or more filtering criteria and provide one or more filtered selected surgical mesh. Filtering criteria can include, but are not limited to, synthetic materials, natural materials (animal tissue), specific types of synthetic or natural materials, absorbable materials, non-absorbable materials, knitted mesh, non-knitted sheet, or any combination thereof.

It can be appreciated that the shear modulus of abdominal tissue 116 can vary in different directions (anisotropy). Thus, it can be desirable for the mesh selection unit 126 to select a mesh that complements the patient's abdominal tissue anisotropy to avoid significant discrepancies (differences) in mechanical properties between the mesh and the abdominal tissue (e.g., differences less than or equal to a predetermined amount or percentage, such as 5%, 10%, etc.). Mechanical properties can include, but are not limited to, tensile strength, compressive strength, elastic modulus (tensile modulus, shear modulus), or any other anatomically relevant mechanical properties, alone or in combination.

Accordingly, in further embodiments, the at least one shear modulus measurement can include a first shear modulus measurement representative of the shear modulus of the patient's abdominal tissue in a first direction and a second shear modulus measurement representative of the shear modulus of the patient's abdominal tissue in a second direction, different from the first direction.

The first and second directions can differ by a predetermined angle. Examples of such predetermined angles can include, but are not limited to, 90 degrees, 45 degrees, 30 degrees, etc. The information stored by the memory can further include a shear modulus in the first direction and the second direction of each surgical mesh of the plurality of surgical meshes. For example, the first direction can be a longitudinal direction extending between the head to feet (e.g., an up-down direction) and a transverse direction extending between opposing sides of the body (e.g., a left-right direction). In another example, the first direction can be approximately parallel to a direction in which the abdominal muscle fibers extend and the second direction can be approximately perpendicular to the direction in which the abdominal muscle fibers extend.

The mesh selection unit 126 can be a program stored in the memory 120 and executed by the processor 122 to select the surgical mesh from the plurality of surgical meshes based upon a comparison of the first shear modulus measurement in the first direction with the shear modulus of the plurality of meshes in the first direction and a comparison of the first shear modulus measurement in the second direction with the shear modulus of the plurality of meshes in the second direction. The selected surgical mesh can have a shear modulus in the first and second directions, respectively, that is greater than the minimum threshold shear modulus for its respective direction or lies within the shear modulus range for its respective direction.

The mesh size/shape unit 130 can be a program stored in the memory 120 and executed by the processor 122 to determine a size and shape of the selected surgical mesh. For example, the at least one shear modulus measurement can include a plurality of shear modulus measurements acquired at different locations of the patient's abdomen within the predetermined distance D from the planned abdominal incision 204. The mesh size/shape unit can identify abdominal locations at which the shear modulus measurement is less than the minimum threshold shear modulus or lies outside of the shear modulus range (method 400, operation 416) and can determine a shape and size for a surgical mesh that covers each of the identified abdominal locations and the planned abdominal incision (method 400, operation 418). The mesh size/shape unit 130 can subsequently output the determined mesh size/shape (method 400, operation 420).

In one aspect, the size and shape of the surgical mesh can be the area A (dashed line envelope) within the predetermined distance D of the planned abdominal incision. In another example, the size and shape of the mesh can be selected to cover available space including and adjacent to the planned abdominal incision (e.g., extending to but not overlapping the coastal margins or fitting into the retromuscular space).

The mesh fixation unit 132 can be configured to determine a type of fixation for the selected surgical mesh to the abdominal tissue 116. In general, surgical mesh fixation is a balance between the benefit of strength of fixation to keep the surgical mesh in place versus the adverse effects of fixing the mesh (e.g., tissue tearing, bleeding, etc.) A surgeon can choose between no fixation and one of a number of types of mesh fixation techniques. Examples of mesh fixation techniques can include penetrating mesh fixation and non-penetrating mesh fixation. Penetrating mesh fixation attaches mesh to local abdominal tissue by permeating the tissue using sutures or tack. Non-penetrating (atraumatic) mesh fixation does not permeate the tissue to attach mesh to local abdominal tissue and can include glue or self-gripping mesh. Materials employed for mesh fixation can be further subdivided into groups such as non-absorbable (permanent) or absorbable and synthetic or natural.

In order for the decision support system 102 to determine an appropriate mesh fixation, the memory 120 can store a look-up table including a plurality of fixation techniques for securing the selected surgical mesh to abdominal tissue 116 of a patient and a range of shear modulus values corresponding to each of the plurality of fixation techniques. The mesh fixation unit 132 can select a surgical mesh from the plurality of surgical meshes based upon a comparison of the respective shear modulus ranges and the at least one shear modulus measurement.

For example, the at least one shear modulus measurement can include a plurality of shear modulus measurements acquired at different locations of the patient's abdomen 202 within the predetermined distance D from the planned abdominal incision 204. The mesh fixation unit 132 can identify the modulus range that includes the at least one shear modulus measurement (method 400, operation 422) and select the fixation technique from the plurality of fixation techniques that corresponds to the identified shear modulus range (method 400, operation 424). The mesh fixation unit 132 can further output the selected fixation technique (method 400, operation 426).

In order to facilitate placement of the selected surgical mesh, the decision support system 102 can further generate one or more composite images 300.

In an embodiment, the system 100 can further include an optical camera 134. The optical camera 134 can be configured to acquire one or more optical images of at least a portion of the patient's abdomen 202 in which SWE measurements are acquired (e.g., optical images within the predetermined distance D of the planned abdominal incision 204).

The composite image 300 can be generated by the processor 122. For example, the processor 122 can receive the one or more optical images from the optical camera 134 and further overlay one or more graphical objects upon the optical image(s). Such graphical objects can include objects representing the planned abdominal incision 204, the determined size/shape of the selected surgical mesh 306, at least one shear modulus measurement, or any combination thereof. The coordinates of respective shear modulus measurement positions can be related to camera coordinates using anatomical markers identified within the optical images.

In alternative embodiments, the optical camera 134 can be omitted from the system 100 and graphical objects representing the planned abdominal incision, the determined size/shape of the selected surgical mesh, and/or at least one shear modulus measurement, can be overlaid upon a schematic anatomical model. For example, the coordinates at which the respective shear wave measurements are acquired (ultrasonic probe coordinates) can be correlated to coordinates on the anatomical model using a predetermined reference location on the patient's anatomy.

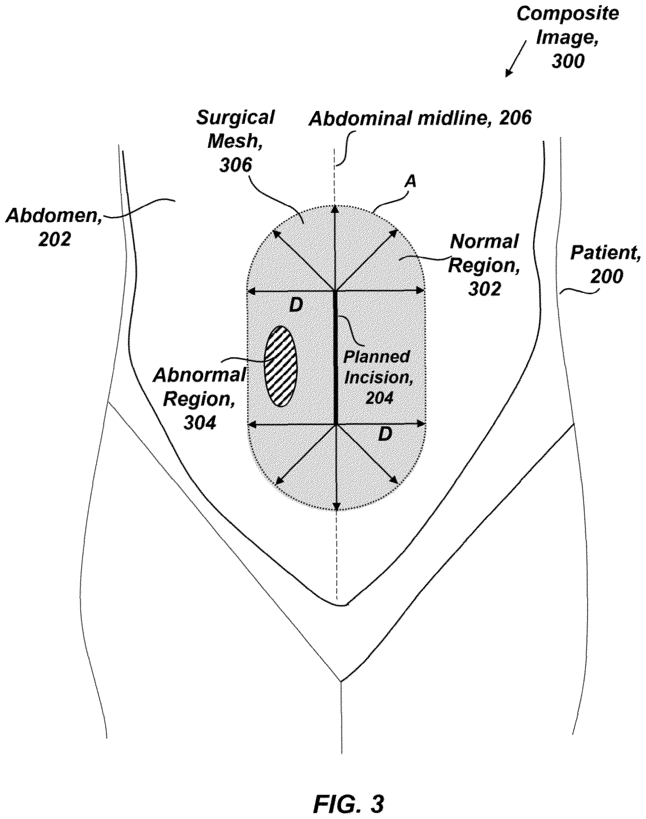

As shown in FIG. 3, a region of shear modulus measurements greater than the minimum threshold shear modulus, or lying within the shear modulus range, is shown in gray shading (normal region) 302. A region of shear modulus measurements less than the minimum threshold shear measurements, or lying outside of the shear modulus range, is shown with a diagonal pattern (abnormal region) 304. The dashed line boundary delineating a predetermined distance D from the planned abdominal incision 204 represents the size and shape of the surgical mesh 306.

Exemplary technical effects of the methods, systems, and devices described herein include, by way of non-limiting example, use of shear wave elastography (SWE) to facilitate pre-operative decisions regarding whether to employ a surgical mesh following laparotomy. When employing a surgical mesh following laparotomy or for ventral hernia repair, decisions regarding selection of a surgical mesh, size and shape of the selected surgical mesh, and/or fixation of the surgical mesh can be tailored to the specific anatomical characteristics of a patient. In this manner, surgeon errors when selecting a surgical mesh can be prevented and improved likelihood of positive outcomes of laparotomy and ventral hernia repair, such as restoration of physiological behavior within the vicinity of a planned abdominal incision and avoiding development/recurrence of an incisional hernia, can be achieved.

Certain exemplary embodiments have been described to provide an overall understanding of the principles of the structure, function, manufacture, and use of the systems, devices, and methods disclosed herein. One or more examples of these embodiments have been illustrated in the accompanying drawings. Those skilled in the art will understand that the systems, devices, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention. Further, in the present disclosure, like-named components of the embodiments generally have similar features, and thus within a particular embodiment each feature of each like-named component is not necessarily fully elaborated upon.

The subject matter described herein can be implemented in analog electronic circuitry, digital electronic circuitry, and/or in computer software, firmware, or hardware, including the structural means disclosed in this specification and structural equivalents thereof, or in combinations of them. The subject matter described herein can be implemented as one or more computer program products, such as one or more computer programs tangibly embodied in an information carrier (e.g., in a machine-readable storage device), or embodied in a propagated signal, for execution by, or to control the operation of, data processing apparatus (e.g., a programmable processor, a computer, or multiple computers). A computer program (also known as a program, software, software application, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file. A program can be stored in a portion of a file that holds other programs or data, in a single file dedicated to the program in question, or in multiple coordinated files (e.g., files that store one or more modules, sub-programs, or portions of code). A computer program can be deployed to be executed on one computer or on multiple computers at one site or distributed across multiple sites and interconnected by a communication network.

The processes and logic flows described in this specification, including the method steps of the subject matter described herein, can be performed by one or more programmable processors executing one or more computer programs to perform functions of the subject matter described herein by operating on input data and generating output. The processes and logic flows can also be performed by, and apparatus of the subject matter described herein can be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processor of any kind of digital computer. Generally, a processor will receive instructions and data from a read-only memory or a random access memory or both. The essential elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, and flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can be implemented on a computer having a display device, e.g., a CRT (cathode ray tube) or LCD (liquid crystal display) monitor, for displaying information to the user and a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The techniques described herein can be implemented using one or more modules or units. As used herein, the term "module" or "unit" refers to computing software, firmware, hardware, and/or various combinations thereof. At a minimum, however, modules or units are not to be interpreted as software that is not implemented on hardware, firmware, or recorded on a non-transitory processor readable recordable storage medium (i.e., modules or units are not software *per se*)*.* Indeed "module" or "unit" is to be interpreted to always include at least some physical, non-transitory hardware such as a part of a processor or computer. Two different modules or units can share the same physical hardware (e.g., two different modules or units can use the same processor and network interface). The modules or units described herein can be combined, integrated, separated, and/or duplicated to support various applications. Also, a function described herein as being performed at a particular module or unit can be performed at one or more other modules or units and/or by one or more other devices instead of or in addition to the function performed at the particular module or unit. Further, the modules or units can be implemented across multiple devices and/or other components local or remote to one another. Additionally, the modules or units can be moved from one device and added to another device, and/or can be included in both devices.

The subject matter described herein can be implemented in a computing system that includes a back-end component (e.g., a data server), a middleware component (e.g., an application server), or a front-end component (e.g., a client computer having a graphical user interface or a web browser through which a user can interact with an implementation of the subject matter described herein), or any combination of such back-end, middleware, and front-end components. The components of the system can be interconnected by any form or medium of digital data communication, e.g., a communication network. Examples of communication networks include a local area network ("LAN") and a wide area network ("WAN"), e.g., the Internet.

Approximating language, as used herein throughout the specification and claims, may be applied to modify any quantitative representation that could permissibly vary without resulting in a change in the basic function to which it is related. Accordingly, a value modified by a term or terms, such as "about," "approximately," and "substantially," are not to be limited to the precise value specified. In at least some instances, the approximating language may correspond to the precision of an instrument for measuring the value. Here and throughout the specification and claims, range limitations may be combined and/or interchanged, such ranges are identified and include all the sub-ranges contained therein unless context or language indicates otherwise.

One skilled in the art will appreciate further features and advantages of the invention based on the above-described embodiments. Accordingly, the present application is not to be limited by what has been particularly shown and described, except as indicated by the appended claims. All publications and references cited herein are expressly incorporated by reference in their entirety.

## Claims

1. A system for pre-operative decision support (102), comprising:
a memory (120) configured to store a minimum threshold shear modulus or shear modulus range for abdominal tissue (116) of a patient (200); and
a processor (122) in communication with the memory (120) and configured to execute one or more programs to perform operations including:
receiving at least one shear modulus measurement acquired by shear wave elastography (SWE) that is representative of a shear modulus of abdominal tissue (116) of the patient (200) within a predetermined distance D of a planned abdominal incision (204);
comparing the at least one shear modulus measurement with the minimum threshold shear modulus or shear modulus range; and
outputting a recommendation regarding use of a surgical mesh (306) following a laparotomy based upon the comparison.

2. The system of claim 1, wherein the one or more programs executed by the processor (122) are further configured to perform operations including outputting a recommendation to use a surgical mesh (306) when the at least one shear modulus measurement is less than the minimum threshold shear modulus or lies outside of the shear modulus range.

3. The system of claim 2, wherein the at least one shear modulus measurement is an average of a plurality of shear modulus measurements acquired at different locations of the patient's abdomen (202) within the predetermined distance D from the planned abdominal incision (204).

4. The system of claim 2,
wherein the at least one shear modulus measurement includes a plurality of shear modulus measurements acquired at different locations of the patient's abdomen (202) within the predetermined distance D from the planned abdominal incision (204); and
wherein the one or more programs executed by the processor (122) are further configured to perform operations including:
identifying abdominal locations at which the at least one shear modulus measurement is less than the minimum threshold shear modulus (304) or lies outside of the shear modulus range;
determining a shape and size for a surgical mesh (306) that covers each of the identified abdominal locations and the planned abdominal incision; and
outputting the determined size and shape;
wherein, optionally, the one or more programs executed by the processor (122) are further configured to perform operations including:
receiving an optical image of the abdomen (202) of the patient (200) within the predetermined distance D of the planned abdominal incision (204);
generating a composite image (300) that overlays, upon the optical image, graphical objects representing the planned abdominal incision (204), the determined size and shape of the surgical mesh (306), and at the least one shear modulus measurement (302, 304); and
outputting the generated composite image (300) for display on a display device (114).

5. The system of claim 1,
wherein the memory (120) is further configured to store a plurality of fixation techniques for securing a surgical mesh (306) to abdominal tissue of a patient (200) and a range of shear modulus values corresponding to each of the plurality of fixation techniques; and
wherein the one or more programs executed by the processor (122) are further configured to perform operations including:
identifying the modulus range that includes the at least one shear modulus measurement;
selecting the fixation technique from the plurality of fixation techniques that corresponds to the identified shear modulus range; and
outputting the selected fixation technique;
wherein, optionally, the selected surgical mesh (306) has a shear modulus in the first and second directions greater than the minimum threshold shear modulus or lies within the shear modulus range.

6. The system of claim 2,
wherein the memory (122) is further configured to store information for each of a plurality of surgical meshes, the information including a shear modulus;
wherein the one or more programs executed by processor (122) are further configured to perform operations including:
selecting a surgical mesh (306) from the plurality of surgical meshes based upon a comparison of the at least one shear modulus of the plurality of surgical meshes with the at least one shear modulus measurement; and
outputting the selected surgical mesh (306).

7. The system of claim 6, wherein the selected surgical mesh (306) has a shear modulus greater than the minimum threshold shear modulus or lies within the shear modulus range.

8. The system of claim 6,
wherein the at least one shear modulus measurement includes a first shear modulus measurement representative of the shear modulus of the patient's abdominal tissue in a first direction and a second shear modulus measurement representative of the shear modulus of the patient's abdominal tissue in a second direction, different from the first direction;
wherein the information stored by the memory (120) includes a shear modulus in the first direction and the second direction of each surgical mesh; and
wherein the one or more programs executed by the processor (122) are further configured to perform operations including selecting the surgical mesh (306) from the plurality of surgical meshes based upon a comparison of the first shear modulus measurement in the first direction with the shear modulus of the plurality of meshes in the first direction and a comparison of the first shear modulus measurement in the second direction with the shear modulus of the plurality of meshes in the second direction.

9. A method for pre-operative decision support, comprising:
receiving, by a processor (122) from a memory (120), a minimum threshold shear modulus or shear modulus range for abdominal tissue of a patient (200);
receiving, by the processor (122), at least one shear modulus measurement acquired by shear wave elastography (SWE) that is representative of a shear modulus of abdominal tissue of the patient (202) within a predetermined distance of a planned abdominal incision;
comparing, by the processor (122), the at least one shear modulus measurement with the minimum threshold shear modulus or shear modulus range; and
outputting by the processor (122), a recommendation regarding use of a surgical mesh (306) following a laparotomy based upon the comparison.

10. The method of claim 9, further comprising, by the processor (122), outputting a recommendation to use a surgical mesh (306) when the at least one shear modulus measurement is less than the minimum threshold shear modulus or lies outside of the shear modulus range.

11. The method of claim 10, wherein the at least one shear modulus measurement is an average of a plurality of shear modulus measurements acquired at different locations of the patient's abdomen (202) within the predetermined distance D from the planned abdominal incision (204).

12. The method of claim 10,
wherein the at least one shear modulus measurement includes a plurality of shear modulus measurements acquired at different locations of the patient's abdomen (202) within the predetermined distance D from the planned abdominal incision (204); and
further comprising, by the processor (122):
identifying abdominal locations at which the at least one shear modulus measurement is less than the minimum threshold shear modulus or lies outside of the shear modulus range;
determining a shape and size for a surgical mesh (306) that covers each of the identified abdominal locations and the planned abdominal incision; and
outputting the determined size and shape; and, optionally,
receiving an optical image of the abdomen (202) of the patient (200) within the predetermined distance D of the planned abdominal incision (204);
generating a composite image that overlays, upon the optical image, graphical objects representing the planned abdominal incision (204), the determined size and shape of the surgical mesh (306), and at the least one shear modulus measurement (302, 304); and
outputting the generated composite image (300) for display on a display device (114).

13. The method of claim 9, further comprising, by the processor (122):
receiving a plurality of fixation techniques for securing a surgical mesh (306) to abdominal tissue of a patient (200) and a range of shear modulus values corresponding to each of the plurality of fixation techniques;
identifying the modulus range that includes the at least one shear modulus measurement;
selecting the fixation technique from the plurality of fixation techniques that corresponds to the identified shear modulus range; and
outputting the selected fixation technique;
wherein, optionally, the selected surgical mesh (306) has a shear modulus in the first and second directions greater than the minimum threshold shear modulus or lies within the shear modulus range.

14. The method of claim 10, further comprising, by the processor (122):
receiving information for each of a plurality of surgical meshes, the information including a shear modulus;
selecting a surgical mesh (306) from the plurality of surgical meshes based upon a comparison of the at least one shear modulus of the plurality of surgical meshes with the at least one shear modulus measurement; and
outputting the selected surgical mesh (306).

15. The method of claim 14, wherein the selected surgical mesh (306) has a shear modulus greater than the minimum threshold shear modulus or lies within the shear modulus range.

16. The method of claim 14, further comprising:
wherein the at least one shear modulus measurement includes a first shear modulus measurement representative of the shear modulus of the patient's abdominal tissue in a first direction and a second shear modulus measurement representative of the shear modulus of the patient's abdominal tissue in a second direction, different from the first direction;
receiving, by the processor (122), a shear modulus in the first direction and the second direction of each surgical mesh; and
selecting, by the processor (122), the surgical mesh (306) from the plurality of surgical meshes based upon a comparison of the first shear modulus measurement in the first direction with the shear modulus of the plurality of meshes in the first direction and a comparison of the first shear modulus measurement in the second direction with the shear modulus of the plurality of meshes in the second direction.
